# EUROPEAN PATENT APPLICATION

(11) **EP 0 806 637 A2**
(43) Date of publication of application: **12.11.1997**
(21) Application number: 97303139.6
(22) Date of filing: 08.05.1997
(51) Int. Cl.: G01F 23/36

(54) **Fuel level indicator systems**

(30) Priority: 09.05.1996 US 17112 P
(71) Applicant: CTS Corporation, Elkhart Indiana 46514-1899 (US)
(72) Inventor: Cooper, Richard O., Bluffton, IN 46714 (US); Holmes, Curtis L., Elkhart, IN 46514 (US); Bloom, Terry R., Middlebury, IN 46540 (US); Zdanys, John, Jr., Elkhart, IN 46514 (US)
(74) Representative: Amann, Hannes Gerhart

(57) **Abstract**

A fuel level indicator system that utilizes a resistor card having an arc-shaped resistive path with a first set of spaced apart conductor lines and an arc-shaped resistive ink material overlying the first set of conductor lines. The first conductor lines are formed at an angle with respect to a radial line drawn from the centre of the resistive path arc. The resistor card also has an arc-shaped continuously solid conductor base and a second set of spaced apart conductor lines extending from the base at an angle with respect to a radial line drawn from the centre of the arc-shaped conductor base. The system includes a wiper assembly having a pair of spaced apart arms. Each arm has a plurality of parallel fingers extending from one end. One of the fingers on a first arm engages a conductor line on the resistive path and a second finger on the first arm engages an adjacent conductor line along the resistive path. A first finger on the second arm engages a conductive line on the conductive path, and a second finger engages an adjacent conductor line on the conductive path. The wiper assembly is constructed and arranged to provide rotational movement along the arc-shaped conductive path and arc-shaped resistive path. The system also has a float with an extending lever that is connected to the wiper assembly to produce rotational movement of the wiper assembly as the float moves.

## Description

This invention relates generally to fuel level indicator systems, and in particular to ceramic resistor card assemblies for use therein.

Ceramic resistor card assemblies have been utilized in fuel level indication systems heretofore. The ceramic material and the inks printed thereon are stable when exposed to harsh elements within a gasoline fuel tank. There are several standard designs.

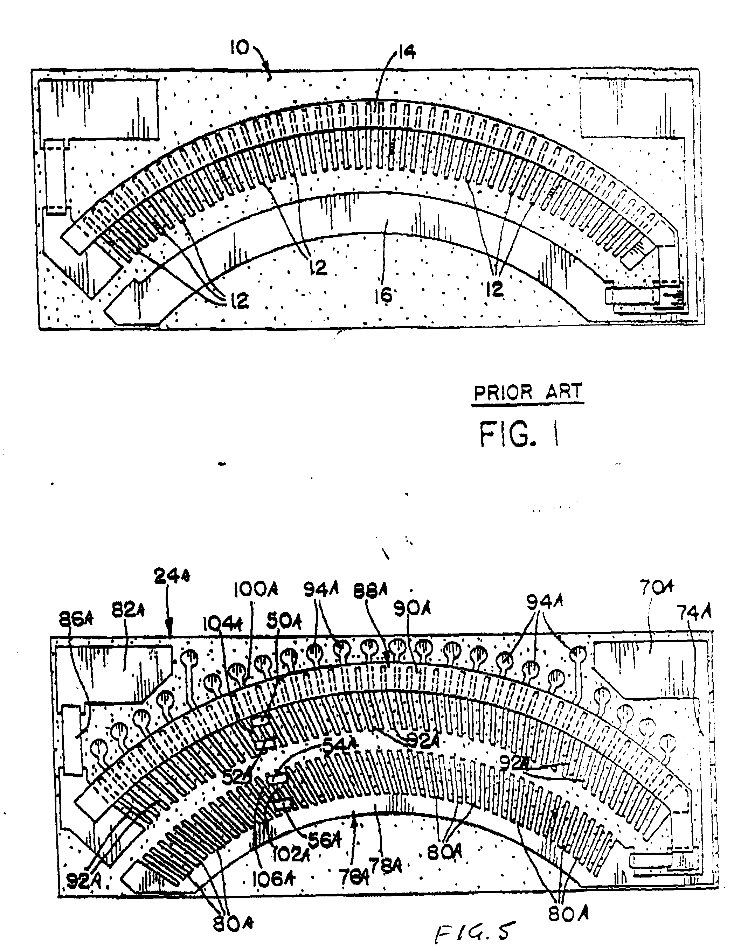

In a first conventional design, contacts located on a wiper assembly form a shorting bar across a resistor on the ceramic card and a conductor on the ceramic card. A prior art ceramic resistor card 10 is shown in Figure 1 of the accompanying drawings. A wiper button makes contact at designated intervals with conductive bars 12 emanating from a resistive ink 14 printed over the conductive bars. A second wiper button rides on a flat continuous conductive path 16 screened onto the ceramic substrate. An alloy of copper, zinc and nickel known as "silver nickel" is used as a material for the contacts due to the relative softness of this material and its relative low cost. Substantial material volume must be utilized to allow for the significant contact wear and thereby to ensure part durability.

In a second conventional design, contacts located on the wiper assembly form a shorting bar across a resistor on a ceramic card and a metal conductor plate. The wiper contact button makes contact at designated intervals with conductive bars emanating from underneath the resistor ink. The second contact button rides on the metal plate.

Both of these conventional designs are susceptible to the buildup of contact resistance. Contact resistance can change the output value of the resistor assembly or in some instances can cause "open circuit" conditions. "Silver nickel" has a tendency to oxidize under environmental conditions found in today's gasoline fuel tanks, and with an increased use of oxygenated fuels. To overcome contact resistance, many designs rely on voltage in the circuit and the "scrubbing" action of the contacts riding across the surface which they wipe on. "Scrubbing" is mechanical abrasion, which is the interaction of the contact surfaces and the force with which the contacts are held against the surfaces. With the designs described above, relatively high forces in the 20 to 40 gram range are used to cause abrasion or wear against the ink/ceramic surface or the metal contact plate. Contact wear is further aggravated by the presence of the fuel. The fuel washes the abrasive surfaces, thereby "renewing" the cutting surfaces of the abrasives in the ceramics and the inks. In contrast, the contact wear under dry conditions creates "smooth" surfaces as surface irregularities are filled in with abraded material. "Burn through" is achieved when sufficient voltage is applied to the circuit to overcome the contact resistance. The designs described above require a voltage which can produce 25 mA to overcome contact resistance on a consistent basis.

The change in electro-mechanical gauges to electronic modules for the display of fuel levels has reduced the voltage provided to the variable resistor from 13.5 volts to 5 volts. Pull-up resistors used in these electronic modules or computers further decrease the voltage so that only 10 mA are available. Since the designs described above require 25 mA to consistently override resistance they cannot be used in these electronic systems.

The recent implementation of onboard diagnostic evaporative emission requirements force the fuel indication system to be more accurate. Any problems resulting from contact resistance may jeopardize the accuracy of the systems and their ability to meet onboard diagnostic requirements.

The present invention seeks to overcome the disadvantages arising in the prior art.

According to the invention, there is provided a fuel level indicator system comprising a resistor card having an arc-shaped resistive path comprising a first set of spaced apart conductor lines and an arc-shaped resistive ink material overlying said first set of conductor lines, and said conductor lines being formed at an angle with respect to a radial line drawn from the centre of the resistive path arc; and an arc-shaped conductive path comprising an arc-shaped continuously solid conductor base and a second set of spaced apart conductor lines extending from said base at an angle with respect to a radial line drawn from the centre of the arc-shaped conductor base.

This invention thereby provides a combustion engine fuel level indication system operable at 10 mA or lower and without any contact resistance buildup.

The present system preferably utilizes a noble metal ink formulation for contacts of an arcuate shaped resistive path and/or conductive path. The contact bar and resistive layer each can have angled conductor lines which reduce the overall volume requirement for the noble metal ink composition. Further, the invention can include a wiper contact having a plurality of spaced apart parallel tanks or fingers positioned so that a first finger engages a first conductor line along the contact bar and the third finger engages an adjacent conductor line along either the contact bar or resistive path to improve the make brake contact.

Thus, an additional feature of the invention is to provide a device that includes a wiper assembly having two sets of spaced apart wiper contacts or fingers. One of the fingers in each set engages a first conductor line and a second finger contacts a second adjacent conductor line. The wiper assembly is constructed and arranged to provide rotational movement along both the arc-shaped conductive and resistive path. A further feature of the invention is to provide a device that has a float and a lever extending from the float connected to the wiper assembly to produce rotational movement of the wiper assembly as the float moves.

The invention is further described with reference to the accompanying drawings, in which:-
Figure 1 is an illustration of a prior art ceramic resistor card for a fuel level indication system;
Figure 2 is a representation of a fuel level detecting system;
Figure 3 is one top view of a ceramic resistor card for use in the system of Figure 1;
Figure 4 is an exploded view of a generally similar fuel level indication system according to the present invention;
Figure 5 is an enlarged view of a ceramic resistor card for a fuel level indication system of Figure 4 as viewed in the direction of arrows 3-3;
Figure 6 is an enlarged view of a contact assembly according to the present invention; and
Figure 7 is a cross-sectional view of a portion of Figure 6 as indicated by line 7-7.

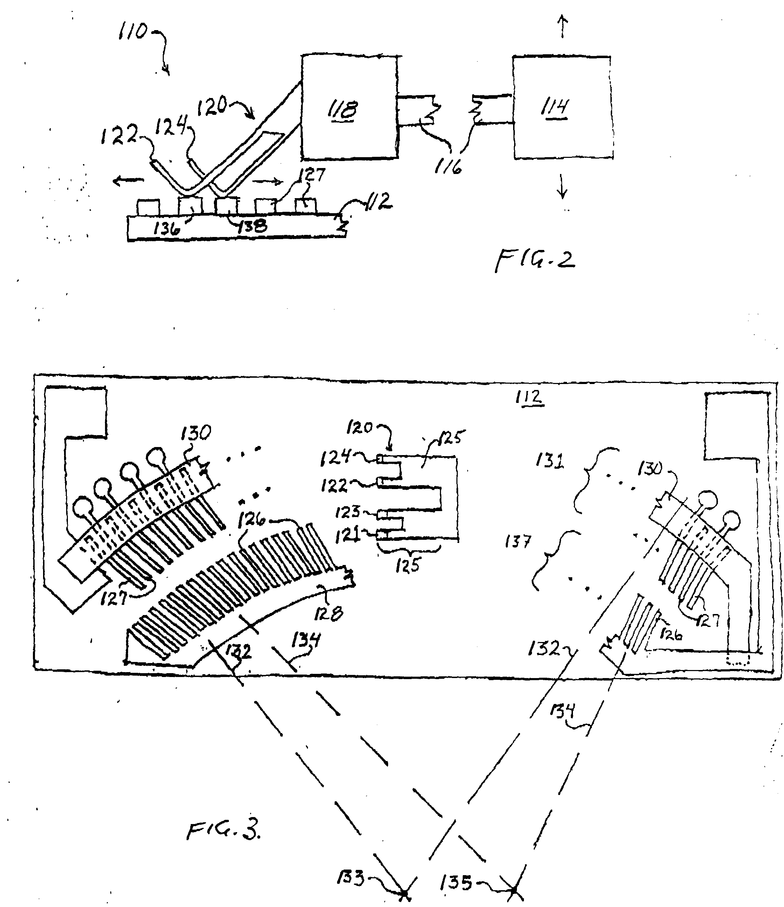

The present invention provides a fuel level detecting system 110 using a unique design of ceramic card 12. Regarding Figure 2 and Figure 3, there is shown fuel float 114 for floating in a fuel tank (not shown). The float 114 is coupled by a coupling 116 to a pivoting wiper linkage 18. Wiper blade assembly 120 is coupled to the pivoting wiper linkage 118 and has two wiper arms 125. Each arm 125 has conductive fingers 121 and 123, and 122 and 124, which are parallel to each other. A ceramic card 112 is used as a base for mounting resistive and conductive traces thereon. Radial lines 132 indicate the pivot point 133 used for determining the parallel arc-shaped design or path of the movable wiper blade assembly 120, arc-shaped resistor trace 130 and arc-shaped solid conductor trace 128. Radial lines 134 indicate the pivot point 135 of radially aligned conductor lines 126 and 127. The resistor trace 130 lies over a portion of each conductor line 127 to form a generally arc-shaped resistive path 131. Conductor lines 126 extend from an arc-shaped continuously solid conductor base 128 to form a generally arc-shaped conductor path 137. Fingers 122 and 124 are positioned to contact conductor lines 127 and fingers 121 and 123 are positioned to contact conductor lines 126 as the wiper blade assembly 120 rotates about point 133.

In operation, as float 114 rises and lowers, coupling 116 moves wiper linkage 118 in a fashion to cause wiper blade assembly 120 to arcuately travel across conductor lines 126 and 127. The moving wiper blade assembly 120 is designed and oriented to have a make-before-break operation, in which the sweeping fingers, i.e. 122 and 124 in Figure 2, make connection with a next conductor line 138 before breaking contact with a currently contacted conductor line 136. Because of the make-before-break design, never will there be an open circuit as a result of non-continuous contact between the fingers and conductor lines as the wiper assembly rotates about point 133.

### Advantages arising are that:-

1) By having the conductor lines 126 and 127 at a different angle than the angle of rotation and orientation for the fingers 121, 122, 123 and 124 a make-before-break connection is created therebetween.
2) The fingers make contact with the conductor lines at an acute angle. A smaller angle of impact will lessen the potential wear on the conductor lines from such impact over thousands of repetitive impacts.
3) The acute angle between the fingers and conductor lines prevents open circuit breaks as the wiper blade assembly sweeps across the ceramic card in the make-before-break design.
4) The use of conductor lines instead of a continuous conductive path means that there is less material used in making the conductor portions, which leads to an overall less expensive part.

It is to be noted that Figure 2 does not illustrate all four contact fingers, but the view looking along a radial line 134 as the fingers contact conductor lines 127. It is further to be noted that resistor trace 130 can be made of resistive ink material. It is also to be noted that radial lines 132 and 134 form an angle to each other.

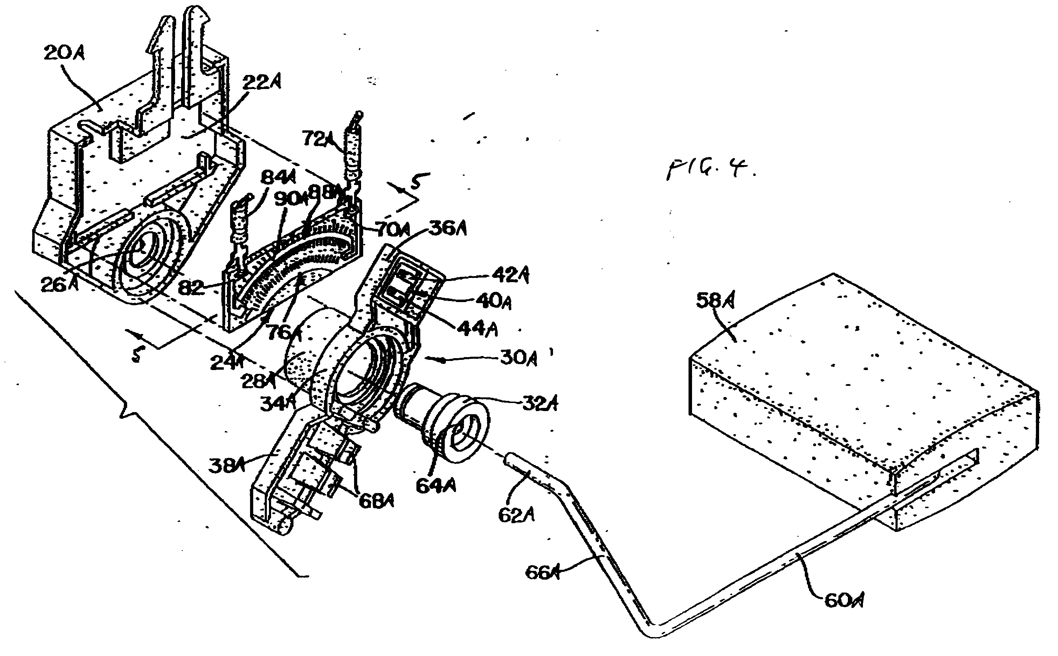

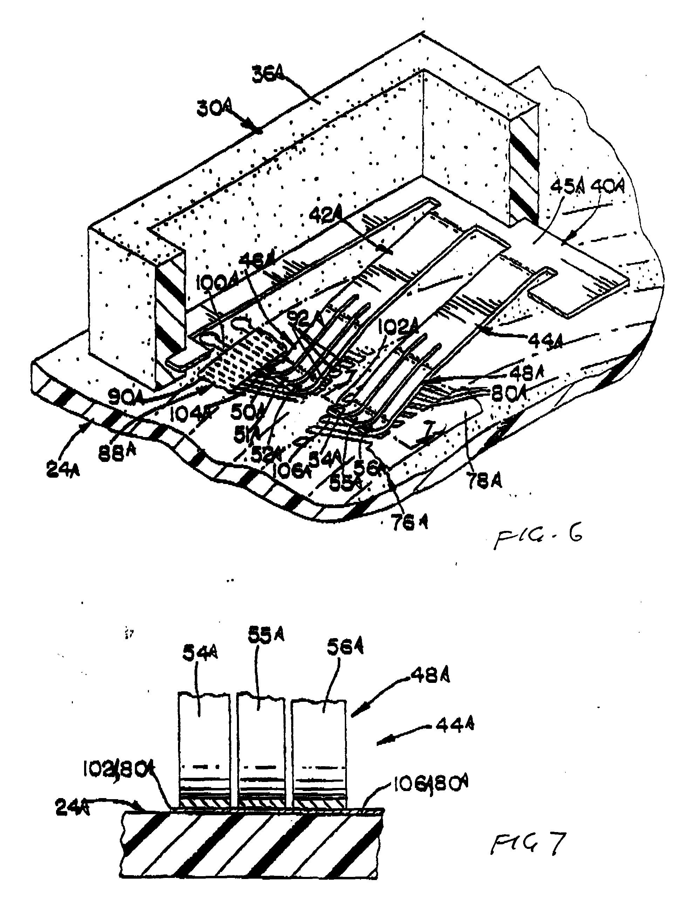

Referring to Figures 4 to 7, there is again illustrated a combustion fuel level indicator system according to the present invention. The fuel level indicator system includes a housing 20A having a recess 22A therein for carrying a resistor card 24A. The housing 20A also has a recess or hole 26A therein for a hollow male portion 28A of a wiper assembly 30A. The wiper assembly 30A is pivotally connected to the housing with the aid of a bushing 32A which extends through the hollow male portion 28A. The wiper assembly includes a body portion 34A from which the hollow male portion 28A extends, an upper head portion 36A and a lower leg portion 38A. The upper head portion carries a contact assembly 40A including a pair of spaced apart parallel arms 42A, 44A connected together by a bridge 45A. Each arm 42A, 44A has a set of fingers 46A, 48A, preferably three, of spaced apart outwardly extending parallel fingers 50A, 51A, 52A and 54A, 55A, 56A. Both sets of fingers 46A, 48A are positioned to travel in an arc-shaped path consistent with the arc-shaped resistance path 88A or arc-shaped conductive path 76A on the ceramic card as described hereafter.

A float 58A is provided for vertical movement in a fuel tank and a lever 60A extends therefrom for engagement in the bushing. The lever extending from the float includes a head portion 62A which extends into a hole 64A in the bushing 32A, and a downwardly extending body portion 66A which is captured by prongs 68A extending outwardly from the leg portion 38A of the wiper assembly. As the float moves up and down due to the changes in fuel level, the lever extending therefrom causes the wiper assembly to rotate.

The resistive card includes a first contact pad 70A having a first wire 72A connected thereto to provide current to the contact pad. A conductive line 74A extends along one edge of the resistive card from the first contact pad 70A to an arc-shaped conductive path 76A. The arc-shaped conductive path 76A includes an arc-shaped continuously solid base portion 78A and a plurality of spaced apart conductor lines 80A extending therefrom at an angle with respect to a radial line drawn from the centre of the base portion's arc. A second contact pad 82A is provided having a second wire 84A secured thereto. A conductive line 86A extends along an opposite side of the resistive card from the second conductor pad to an arc-shaped resistive path 88A. The arc-shaped resistive path includes an arc-shaped resistive ink pattern 90A overlying a plurality of spaced apart conductor lines 92A formed at a radial line drawn from the centre of the arc of the resistive ink pattern. A plurality of small contact pads 94A extend from the selective conductor lines 92A of the resistive path for purposes of laser trimming the resistive path.

As the float 58A moves up and down with respect to changing fuel levels, the lever 60A extending from the float causes the wiper assembly 30A to rotate. As the head portion 36A of the wiper assembly rotates, the first set of tangs or fingers 46A on the first contact arm 42A engage the conductor lines 92A of the resistive path 88A and the second set of tangs or fingers 48A on the second contact arm 44A engage the conductor lines 80A of the arc-shaped conductive path 76A. With respect to both the resistive path 88A and conductive path 76A, a top finger 50A, 54A of the contact arm 42A, 44A will engage one of the conductor lines 100A, 102A and a third finger 52A, 56A (parallel to the first finger) on the same arm will contact an adjacent conductor line 104A, 106A respectively. Thus, because the conductor lines 80A, 92A are formed at an angle with respect to a radial line drawn from the centre of the arc of the resistive pattern or conductive base portion respectively, and the fingers 50A and 52A, 54A and 56A on each arm are parallel, the contact arm is simultaneously contacting adjacent conductor lines thus minimising or eliminating any problem associated with make brake contact of prior art resistor cards. Preferably, each set of fingers 46A, 48A are curled so that the finger can move smoothly over the elevated conductor lines 80A, 92A. The fingers 50A - 56A of a contact are made from an alloy including preferably 45% of palladium and/or platinum, 38% silver and 17% copper by weight. The fingers are 3/1000 of an inch thick or less. These high content precious metal contacts are extremely resistant to oxidation allowing the system to operate at 10 mA.

Preferably the conductor lines 80A, 92A of both the resistive path and the conductive path are formed from a conductive ink composition comprising at least 35 weight percent silver and up to 65 weight percent silver.

Because spaced apart conductor lines are used for each path, the wiper contact assembly 40A wears less. This is because the sets of contact fingers 46A, 48A come in contact with less material during the wiping operation.

Further, since the conductive path 76A is made from an ink containing a relatively high concentration of noble metals, the use of spaced apart conductor lines reduces the cost of the resistor card compared to a continuous wide solid conductor line or path of the prior art. It is also believed that the conductive path's ink having a relatively high concentration of silver allows silver to migrate to the surface. The silver on the surface of the conductive path provides a "softer" more ductile surface for the contacts to ride on and thus reduces the wear of the contact assembly 40A.

In operation, current is provided through a wire 72A to the first contact pad 70A and flows therefrom to the conductive path 74A printed on the resistor card. The set of fingers 48A on the second arm 44A of the contact wiper assembly engages at least two of the conductor lines 102A, 106A on the conductive path 76A. The current then flows across the contact 40A through the first arm 42A and first set of fingers 46A to at least two conductor lines 100A, 104A on the resistive path 88A. As the fuel float 58A moves with the fuel level, the wiper assembly rotates along the conductive and resistive paths. Consequently, a variable resistance to current is provided dependent upon where the contact engages the resistive path. As a result, the present fuel level indicator system can be operated at 10 mA or lower as compared to at least 25 mA in the prior art.

## Claims

1. A fuel level indicator system comprising a resistor card having an arc-shaped resistive path comprising a first set of spaced apart conductor lines and an arc-shaped resistive ink material overlying said first set of conductor lines, and said conductor lines being formed at an angle with respect to a radial line drawn from the centre of the resistive path arc; and an arc-shaped conductive path comprising an arc-shaped continuously solid conductor base and a second set of spaced apart conductor lines extending from said base at an angle with respect to a radial line drawn from the centre of the arc-shaped conductor base.

2. A fuel level indicator system as claimed in claim 1 further comprising:
a wiper assembly having a pair of spaced apart arms, each arm having a plurality of parallel fingers extending from one end and constructed and arranged so that one of said fingers on a first arm engages a conductor line on the resistive path and a second finger on said first arm engages an adjacent conductor line along said resistive path; and a first finger on said second arm engages a conductive line on said conductive path; and
a second finger engaging an adjacent conductor line on said conductive path, said wiper assembly being constructed and arranged to provide rotational movement along the arc-shaped conductive path and arc-shaped resistive path;
a float having a lever extending therefrom and connected to said wiper assembly to produce rotational movement of the wiper assembly associated with movement of the float.

3. A fuel level indicator system as claimed in claim 1 or claim 2, wherein said conductive path comprises at least 35 weight percent silver.

4. A fuel level indicator system as claimed in any of claims 1 to 3, adapted to operate at 10 mA or less.

5. A fuel level indicator system as claimed in any of claims 1 to 4, wherein said fingers comprise at least 35 weight percent silver, at least 45 weight percent of a material selected from the group consisting of palladium and platinum, and mixtures thereof, and copper.
